# EUROPEAN PATENT APPLICATION

(11) **EP 2 368 549 A1**
(43) Date of publication of application: **28.09.2011**
(21) Application number: 10305303.9
(22) Date of filing: 25.03.2010
(51) Int. Cl.: A61K 31/415

(54) **Treating hand-foot syndrome and related pathologies using clonidine or its derivatives**

(71) Applicant: BioAlliance Pharma, 75015 Paris (FR)
(72) Inventor: The designation of the inventor has not yet been filed
(74) Representative: Jacobson, Claude

(57) **Abstract**

The present invention relates to clonidine or its derivatives for the treatment of Hand-Foot Syndrome (HFS) or related pathologies. A pharmaceutical composition comprising at least clonidine or its derivatives for preventing or treating HFS or related pathologies is another embodiment of the invention. In a further aspect, the present invention provides topical formulation for the application of clonidine or its derivatives that can be used on cutaneous surfaces for preventing or treating HFS or related pathologies.

## Description

### TECHNICAL FIELD OF THE INVENTION

The present invention relates to clonidine or its derivatives for the treatment of Hand-Foot Syndrome (HFS) or related pathologies. A pharmaceutical composition comprising at least clonidine or its derivatives for preventing or treating HFS or related pathologies is another embodiment of the invention. In a further aspect, the present invention provides topical formulation for the application of clonidine or its derivatives that can be used on cutaneous surfaces for preventing or treating HFS or related pathologies.

### BACKGROUND OF THE INVENTION

The skin, belonging to the epithelium tissue, is a soft outer covering of an animal, in particular a vertebrate. The adjective cutaneous literally means "of the skin" (from Latin cutis, skin).

The skin is made of two layers: (i) the epidermis, (ii) the dermis. Another layer, named hypodermis is not part of the skin but is closely connected to it. The epidermis consists primarily of stratified epithelial cells, or keratinocytes, organized in a basal layer, a spinous layer, a granular layer and the stratum corneum (SC). The SC consists of terminally differentiated lipid-depleted keratinocytes that have replaced their plasma membrane with a tough insoluble macromolecular layer called cornified envelope. The stratum corneum serves an important barrier function by keeping molecules from passing into and out of the skin.

The dermis is the layer of skin beneath the epidermis that consists of connective tissue and cushions the body from stress and strain. The dermis is tightly connected to the epidermis by a basement membrane. It also harbors many Mechanoreceptor/nerve endings that provide the sense of touch and heat. It contains the hair follicles, sweat glands, sebaceous glands, apocrine glands, lymphatic vessels and blood vessels. The dermis is structurally divided into two areas: a superficial area adjacent to the epidermis, called the papillary region, and a deep thicker area known as the reticular region.

The hypodermis is not part of the skin, and lies below the dermis. Its purpose is to attach the skin to underlying bone and muscle as well as supplying it with blood vessels and nerves. It consists of loose connective tissue and elastin. The main cell types are fibroblasts, macrophages and adipocytes (the hypodermis contains 50% of body fat). Fat serves as padding and insulation for the body.

The main function of skin is to provide protection as a physical barrier to the entry of foreign materials that include irritants, allergens, pathogens, and to regulate the loss of water. This function is mainly allowed by the SC.

As also belonging to the epithelium tissue, mucosa (or mucous membranes) is different from the skin. Mucosa lines various body cavities that are exposed to the external environment and internal organs. Mucosa is composed of non keratinised stratified squamous epithelium and thus does not present any SC.

The skin can be subject to a wide variety of injuries, trauma or diseases that cause many kinds of inflammation. Inflammation is a complex biological response of vascular tissues and immune cells to harmful stimuli, such as pathogens, damaged cells, or irritants, like for instance chemotherapeutic agents or radiotherapy. It is characterized by five signs: rubor (redness), calor (increased heat), tumor (swelling), dolor (pain), and/or functio laesa (loss of function). Many kind of inflammatory diseases of the skin can appear.

For instance, Palmar erythema (PE) is characterized by redness of palm and skin due to inflammation. It is associated with various physiological as well as pathological changes, the principal one of which is portal hypertension. It is also seen in patients with liver dysfunction, such as chronic liver disease, and also in pregnant women. Palmar erythema may also be the result of thyrotoxicosis and dermatoses such as eczema or psoriasis, and is related to deep telangiectasias. (Serrao R., 2007). Unfortunately, PE is not sufficiently managed by the current therapies.

Inflammation diseases of the skin can in particular occur in the case of anticancer radiotherapy and/or chemotherapy. One frequent complication of chemotherapy is the damage called Hand-Foot Syndrome (HFS), also known as palmer-plantar erythrodysesthesia (PPE) or Hand-Foot Skin Reaction (HFSR) . It is indeed a potential side-effect induced by cancer chemotherapy (Nagore E et al. Am J Clin Dermatol 2000, 1 pp. 225-34). Tissues affected by HFS exhibit inflammatory changes such as dilated blood vessels, oedema and white blood cell infiltration (Abushullaih S et al. Cancer Invest 2002; 20: 3-10; Lipworth AD et al. Oncology 2009; 77:257-271).

HFS can be caused by several types of cytotoxic drugs or biological therapies used to treat cancer including, but not limiting to, capecitabine, 5-fluorouracil (5FU), continuous infusion doxorubicin, pegylated liposomal doxorubicin (PLD), cytarabine, docetaxel, gemcitabine, vinorelbine, floxuridine or idaribicin (Walko CM et al., Clin Ther 2005; 27: 23-44). High doses of traditional chemotherapeutic agents such as 5FU, doxorubicin, and cytarabin remain the primary causes of HFS for nearly 2 decades (Nagore E. et al. Am J Clin Dermatol 2000, 1 pp. 225-234; Susser WS. et al. J Am Acad Dermatol,1999; 40 pp. 367-398). However, in the past 10 years, PLD and capecitabine, closely related variants of their respective predecessors, doxorubicin and 5-FU, may each cause HFS in approximately half of patients receiving therapeutic doses.

Capecitabine (Xeloda ; F. Hoffmann La-Roche, Basel, Switzerland) is an orally administered precursor of 5FU which is tending to replace 5FU in the treatment of a number of malignancies including breast cancer and gastrointestinal (GI) cancers such as colorectal, gastric, pancreatic and others (Walko CM et al., Clin Ther 2005; 27: 23-44). Capecitabine (N4-pentyloxycarbonyl-5'-deoxy-5-fluorocytidine) was designed to generate 5FU preferentially in tumour tissue compared with healthy tissue (Miwa M and al., Eur J Can 1998; 34: 1274-81). Capecitabine is metabolised to 5FU via a three-step enzymatic process. In the first step, Capecitabine is hydrolysed by carboxylesterase in the liver to the intermediate 5'-DFCR. In the second step, 5'-DFCR is converted to 5'-deoxy-5-fluorouridine (5'-DFUR) by cytidine deaminase, which is highly active in the liver and tumour tissue. In the third step, 5'-DFUR is converted to 5FU by thymidine phosphorylase (TP), which is present in tumour tissue, resulting in the release of 5FU preferentially in tumour tissue. TP occurs at levels 3-10 times as high in tumour cells than in healthy tissues. This can enable selective drug activation of 5FU at the tumour site and limit systemic toxicity (Van Cutsem E et al., J Clin Oncol 2001; 19:4097-106).

Capecitabine is generally well-tolerated and has an improved tolerability profile compared with bolus 5FU (Walko CM et al., Clin Ther 2005; 27: 23-44). The most common dose-limiting adverse events are hand-foot syndrome (HFS), diarrhoa and hyperbilirubinemia. Other frequent adverse events include fatigue/weakness, abdominal pain and other gastro-intestinal effects such as nausea/vomiting and stomatitis/mucositis. Compared with bolus 5FU, Capecitabine is associated with less stomatitis, alopecia, diarrhoea, nausea and neutropenia (Gressett SM et al., J Oncol Pharm Pract 2006; 12: 131-41). However Capecitabine is associated with more HFS than bolus 5FU. Indeed, the overall incidence of HFS with capecitabine reported in clinical trials of breast or colorectal cancer has been around 50%, with 17% of patients reporting a severe form, well-known by the skilled person as "grade 3 (Walko CM et al. Clin Ther 2005 27: 23-44).

More recently, the multi-Tyrosine Kinase Inhibitors (TKls) sorafenib and sunitinib, two novel rationally designed and orally dosed drugs active against a variety of cancers, have emerged as significant causes symptoms of HFS, calling Hand Foot Skin Reaction (HFSR). As HFS, HFSR manifests with tender palmoplantar lesions especially in areas of trauma or friction, leading to significant alteration in quality of life (Lacouture ME. et al., 2008, Annals of Oncology, 19 pp1955-1961).

The symptoms of HFS have been well-studied and include numbness, dysesthesia/paresthesia, oedema, tingling, erythma, painless swelling or discomfort and, in more severe cases, blisters, ulceration, desquamation or severe pain on the palms of the hands and/or the soles of the feet (Heo YS and al., J Clin Pharmacol 2004; 44: 1166-72). The majority of patients showing dysesthesia usually present also a tingling sensation of the palms and soles, which may progress to burning pain with swelling and erythema in 3-4 days. The hands are usually more affected than the feet and can be the only area affected. More severe HFS can be very uncomfortable and can interfere with patients' everyday activities. Moreover, it can necessitate a reduction in the dose of the chemotherapeutic agent, treatment interruption or withdrawal.

Three histopathologic features predominate in the state of the art for both traditional chemotherapy-associated HFS and multi-TKI-associated HFS (HFSR): (i) dyskeratotic keratinocytes at various stages of necrosis, (ii) basal layer vacuolar degeneration, and (iii) dermal edema with mononuclear perivascular infiltrate (Lipworth AD et al. Oncology 2009; 77:257-271).

During HFS and HFSR, histological changes can be correlated with clinical severity (Hueso L. et al. 2008, actas Dermosifiliogr, 99 pp281-290) :
- grade 1, characterized by minimal skin changes or dermatitis without pain, showed capillary dilatation and basal cell abnormalities;
- grade 2, characterized by skin changes (peeling, blisters, bleeding, edema) or pain but not interfering with functions/ Capillary dilatation with papillary dermal edema, interphase dermatitis, basal layer degeneration, and scattered necrotic keratinocytes are observed.
- grade 3, characterized by ulcerative dermatitis or skin changes interfering with functions, revealed abundant necrotic keratinocytes, edema, epidermal separation, and interphase dermatitis.

The current treatments to manage HFS or HFSR concern non specific symptoms relief. Skin care creams containing ingredients such as petroleum jelly, lanolin, or lactic acid may be advice by doctors. Doctors can also recommend topical or oral corticoids or vitamin B6 (pyridoxine) (Mrozek-Orlowski M and al. Oncol Nurs. Forum 1999, 26(4) pp 753-62; Lorusso D. et al. 2007, Ann Oncol, 18 pp1159-1164).

Other treatments aiming to target the causative mechanisms of HFS are under development. The enzymes DihydroPyrimidine Dehydrogenase (DPD) or Thymidine Phosphorylase (TP) have been suggested to explain the occurrence of HFS (Yamashita and al. J Toxicol Sci 2004 ; Milano G. et al. Br J Clin Pharmacol 2008). Hence, inhibitors of TP and/or DPD are developed for the treatment of HFS.

These developments are based on topical formulation. For instance, 6-substituted 5-fluorouracil derivatives (Kalman et al. Nucleosodes, Nucleotides, and Nucleic acids, 2005) or Allopurinol (W02007138103) have been previously described as TP inhibitors. Uracil (US6979688) or uracil derivatives, like eniluracil (W02009100367), are examples of DPD inhibitor currently developed for the treatment of HFS.

However, it remains a need for new medications dedicated to the treatment of HFS or HFSR and preferably of its causative mechanisms.

HFS has an indirect effect on the efficacy of cancer treatment. So there is a huge need for a HFS state management (continuous, coordinated, evolutionary process that seeks to manage and improve the health status of a carefully defined patient population over the entire course of a disease). This process includes HFS or HFSR prevention efforts as well as patient management after HFS or HFSR has developed.

The causative mechanisms of HFS or HFSR are not well understood. Direct toxicity of chemotherapeutic agents against epithelium remains the most commonly accepted theory of pathogenesis (Lipworth AD et al. Oncology 2009; 77: 257-271). It is expected that anticancer radiotherapy and/or chemotherapy generate reactive oxygen species (ROS) that damage connective tissue, DNA, cell membranes, stimulate macrophages, and trigger a cascade of critical biologic mechanisms and molecular pathways, leading to inflammation. The resulting inflamed tissue has increased capillary permeability which causes extravasation of the drug and retention effect Lyass O. et al. Cancer 2000; 89(5): 1037-1047). This phenomenon increases localization of cytotoxic chemotherapeutics agents into inflammatory skin, hence get worse local toxicity. All these deleterious events occur in the dermis and lead to epidermal damage through decreased release of cell survival factors, with subsequent keratinocytes apoptosis, and increased release of pro-inflammatory chemokines, with ensuing clinical symptoms.

Thus the causative mechanism of HFS or HFSR can be schematically divided in three steps. In a first step, anticancer radiotherapeutic and/or chemotherapeutic agent trigger inflammation in the dermis. In a second step, said inflammation increases the capillary permeability and causes retention of the drug, enhancing its local toxicity. In a third step said increased local toxicity induces keratinocytes apoptosis and pro-inflammatory chemokines in the epidermis, ensuing clinical symptoms.

Clonidine hydrochloride, also known as catapressan®, is described as an alpha-2 adrenergic receptor agonist that stimulates the alpha-2 adrenoreceptors in the brain stem. It is generally used as an anti-hypertensive agent. Clonidine belongs to the group of phenyl imidazol-amine and has the following formula:

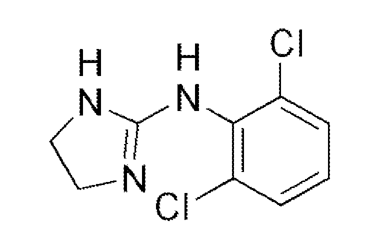

Recently, new uses for clonidine are under consideration for a variety of ailments. For example, clonidine can be used to treat insomnia or menopausal symptoms. In other applications clonidine is used to treat attention-deficiency hyperactivity disorder, as well as Tourette syndrome.

It has been surprisingly shown that clonidine and its derivatives enable to block the three steps of the causative mechanisms leading to HFS. Hence, clonidine and its derivatives are particularly appropriate for the treatment of HFS or related pathologies. Additionally, clonidine and its derivatives are known to reduce the sweating (Delaunay L et al. Anesth Analg. 1996 83(4):844-8). The sweat has been described to function as a carrier of chemotherapeutic agents to the skin surface (Jacobi U. et al. Annal Oncol. 2005 (16):1210-11). After excretion on the skin surface, the sweat containing the drug may penetrate into the stratum corneum. Clonidine is suitable to limit this phenomenon, and reduce HFS.

### SUMMARY OF THE INVENTION

The present invention discloses a new and inventive therapeutic application of Clonidine or its derivatives. Indeed, it has been shown that clonidine and its derivatives present a surprisingly efficacy in the treatment of HFS or related pathologies.

Moreover, as clonidine or its derivatives are able to target all of the HFS or related pathologies causative mechanisms, they represent very promising treatment of said ailments.

A first embodiment of the present invention is thus clonidine or its derivatives for the treatment of HFS or related pathologies. Clonidine or its derivatives are usable in the form of a base or a pharmaceutically acceptable salt. A preferred pharmaceutically acceptable salt is clonidine hydrochloride.

Another embodiment of the invention relates to a pharmaceutical composition comprising clonidine or its derivatives or one pharmaceutically acceptable salt thereof for the treatment of HFS or related pathologies.

Another aspect of the present invention is clonidine or its derivatives associated with other active ingredient for the treatment of HFS or related pathologies. Said other active ingredients are selected from the group consisting of:
- TP inhibitors like, but not limited to 6-substituted 5-fluorouracil derivatives or Allopurinol, ...,
- DPD inhibitors like, but not limited to uracil or uracil derivatives (such as eniluracil), ...,
- Anti-inflammatories like, but not limited to cyclo-oxygenase-2 Inhibitors (such as celecoxib, indomethacin and ibuprofen), corticosteroids (such as dexamethasone, clobetasol 17 propionate, betamethasone, ...),
- Differentiation agents like, but not limited to retinoids, vitamin D or its derivatives, glitazones or fibrates (which stimulate peroxisome proliferation activation receptors (PPAR)), ...,
- Vasoconstrictors like, but not limited to nicotine, caffeine and other stimulants, ...,
- antiperspirant like, but not limited to aluminium or zirconium salts, anticholinergics (such as glycopyrrolate), carbonic anhydrase inhibitors (such as topiramate), ...,
- antiangiogenics like, but not limited to tacrolimus, pimecrolimus or anti-VEGF,

A further aspect of the invention relates to pharmaceutical compositions comprising clonidine, its derivatives or one pharmaceutically acceptable salt thereof, as a first active ingredient and other active ingredients selected from the group consisting of:
- TP inhibitors like, but not limited to 6-substituted 5-fluorouracil derivatives or Allopurinol, ...,
- DPD inhibitors like, but not limited to uracil or uracil derivatives (such as eniluracil), ...,
- Anti-inflammatories like, but not limited to cyclo-oxygenase-2 Inhibitors (such as celecoxib, indomethacin and ibuprofen), corticosteroids (such as dexamethasone, clobetasol 17 propionate, betamethasone, ...),
- Differentiation agents like, but not limited to retinoids, vitamin D or its derivatives, glitazones or fibrates (which stimulate peroxisome proliferation activation receptors (PPAR)), ...,
- Vasoconstrictors like, but not limited to nicotine, caffeine and other stimulants, ...,
- antiperspirant like, but not limited to aluminium or zirconium salts, anticholinergics (such as glycopyrrolate), carbonic anhydrase inhibitors (such as topiramate), ...,
- antiangiogenics like, but not limited to tacrolimus, pimecrolimus or anti-VEGF,

Said pharmaceutical compositions can be administered in a suitable formulation to mammals by topical or systemic administration. In a preferred aspect, said pharmaceutical composition is appropriate for topical administration like, but not limited to, adhesives (film forming formulation, polymer patches), gels, ointments, emulsions (lotions, creams), solutions, suspensions (nanoparticles and microparticles of various compositions) or foams.

Said compositions may constitute a whole part of a HFS state management. Quick drug action is needed when facing with the occurrence of HFS or established HFS. Sustained drug action is needed in case of prevention and in order to avoid relapse.

Surprisingly, quick therapeutic action of said compositions with limited systemic in the case of HFS has been obtained by an inventive approach consisting of applying a topical formulation with a high drug concentration (whatever the thermodynamic state: dispersed, solubilized, or supersaturated) and then removing drug from the skin after a short contact period (less than about 1 h) by washing it off.

Prevention and sustainment care can be obtained by any formulations like, but not limited to adhesives (film forming formulation, polymer patches), gels, ointments, emulsions (lotions, creams), solutions, or foams.

It is known that skin area subjected to repeated contact pressure is prone to occurrence of HFS. In order to increase the efficacy by releasing clonidine only on the skin area involved in HFS appearance, release of clonidine set off by pressure or friction is desirable.

The compounds are used topically at a concentration generally of from 0.001% to 50%, by weight, preferably 0.001% to 10% by weight and more preferably from 0.01 % to 5% by weight, relative to the total weight of the composition.

The compositions may be administered by any means effective to apply the composition to the skin. The compositions may be applied manually, with an applicator or as a spray. The compositions may be incorporated in any woven, nonwoven tissue or bandage.

Formulations may be prepared using pharmaceutically acceptable excipients composed of materials that are considered safe and effective and may be administered to an individual without causing undesirable biological side effects or unwanted interactions. The excipients are all components present in the pharmaceutical formulation other than the active ingredient or ingredients. As generally used herein "excipient" include, but is not limited to surfactants, gelling agents, oils, buffers, water-miscible solvents, preservatives, antioxidants, chelating agents, moisturizers and skin penetrating agent.

The invention has also as embodiment a process for producing a pharmaceutical composition comprising at least clonidine or its derivatives as active principle.

In a further embodiment, the present invention relates to a process for treating HFS or related pathologies in a mammal in need thereof, said process comprising administering to said mammal a pharmaceutically acceptable amount of clonidine or its derivatives, associated or not with other active ingredient selected from the group consisting of:
- TP inhibitors like, but not limited to 6-substituted 5-fluorouracil derivatives or Allopurinol, ...,
- DPD inhibitors like, but not limited to uracil or uracil derivatives (such as eniluracil), ...,
- Anti-inflammatories like, but not limited to cyclo-oxygenase-2 Inhibitors (such as celecoxib, indomethacin and ibuprofen), corticosteroids (such as dexamethasone, clobetasol 17 propionate, betamethasone, ...),
- Differentiation agents like, but not limited to retinoids, vitamin D or its derivatives, glitazones or fibrates (which stimulate peroxisome proliferation activation receptors (PPAR)), ...,
- Vasoconstrictors like, but not limited to nicotine, caffeine and other stimulants, ...,
- antiperspirant like, but not limited to aluminium or zirconium salts, anticholinergics (such as glycopyrrolate), carbonic anhydrase inhibitors (such as topiramate), ...,
- antiangiogenics like, but not limited to tacrolimus, pimecrolimus or anti-VEGF,

Another embodiment of the invention relates to the use of clonidine or its derivatives associated or not with other active ingredient selected from the group consisting of TP inhibitors (like, but not limited to 6-substituted 5-fluorouracil derivatives or Allopurinol),
- TP inhibitors like, but not limited to 6-substituted 5-fluorouracil derivatives or Allopurinol, ...,
- DPD inhibitors like, but not limited to uracil or uracil derivatives (such as eniluracil), ...,
- Anti-inflammatories like, but not limited to cyclo-oxygenase-2 Inhibitors (such as celecoxib, indomethacin and ibuprofen), corticosteroids (such as dexamethasone, clobetasol 17 propionate, betamethasone, ...),
- Differentiation agents like, but not limited to retinoids, vitamin D or its derivatives, glitazones or fibrates (which stimulate peroxisome proliferation activation receptors (PPAR)), ...,
- Vasoconstrictors like, but not limited to nicotine, caffeine and other stimulants, ...,
- antiperspirant like, but not limited to aluminium or zirconium salts, anticholinergics (such as glycopyrrolate), carbonic anhydrase inhibitors (such as topiramate), ...,
- antiangiogenics like, but not limited to tacrolimus, pimecrolimus or anti-VEGF,
for producing a medication for the treatment of HFS or related pathologies.

### DESCRIPTION OF THE PREFERRED EMBODIMENTS OF THE INVENTION

The term "HFS or related pathologies" encompasses Hand-Foot Syndrome (HFS), Hand-Foot Skin Reaction (HFSR) or Palmar Erythema (PE),

The term "mammal" encompasses any of various warm-blooded vertebrate animals of the class Mammalia, including humans, characterized by a covering of hair on the skin and, in the female, milk-producing mammary glands for nourishing the young.

The term "treatment", "treating" or "treat", means preventing, alleviating, reversing, inhibiting the progress of, or cure HFS or related pathologies.

The term "prevent" or "preventing" means that treatment is started prior to the beginning of HFS or related pathologies, thus hindering the onset of said HFS or related pathologies.

A "chemotherapy" means the use of cytotoxic drugs or biological therapies to treat cancer or the combination of these drugs. As used herein, the term "cytotoxic drugs", also named "chemotherapeutic agent", refers to cytotoxic drugs well known by the skilled person. For instance, such chemotherapeutic agents are selected from the group consisting of, but not limiting to, capecitabine, 5-fluorouracil (5FU), doxorubicin, liposomal doxorubicin, cytarabine, docetaxel, gemcitabine, vinorelbine, floxuridine or idaribicin.

The term clonidine encompasses all forms of N-(2,6-dichlorophenyl)-4,5-dihydro-1H-imidazol-2-amine and includes clonidine hydrochloride formulations, as well as the pharmaceutically acceptable salts.

The term clonidine derivatives means clonidine and its pharmaceutical salts or chemically similar derivatives belonging to the group of phenyl imidazol-amine or their prodrugs.

The term prodrug means a pharmacological substance (drug) administered in an inactive (or significantly less active) form which undergoes a chemical transformation *in vivo* to release the active drug.

Chemically similar derivatives of clonidine can also be used in the pharmaceutical composition of the present invention such as 3,5-dichloro-4-(imidazolidin-2-ylideneamino)benzyl alcohol, 3, 5-dichloro-4-(1,3-diisobutyrylimidazolidin-2-ylideneamino)benzyl isobutyrate and ethyl 3,5-dichloro-4-(1-isoburtyrylimidazolidin-2 benzoate. Para-substituted derivatives of clonidine such as amino-,diethylamino-, ethylamino- acetamido-bromoacetamido- N-chlorethyl-N-methyl-amino- and N-β-chloroethyl-N-methylaminomethyl.

Formulations may be prepared using pharmaceutically acceptable excipients composed of materials that are considered safe and effective and may be administered to an individual without causing undesirable biological side effects or unwanted interactions. The excipients are all components present in the pharmaceutical formulation other than the active ingredient or ingredients. As generally used herein "excipient" include, but is not limited to surfactants, , gelling agents, oils, buffers, water-miscible solvents, preservatives, antioxidants, chelating agents, buffers, solvents and preservatives, moisturizers and skin penetrating agent.

A skin penetrating solvent may also or alternatively comprise one or more compounds selected from the group consisting of dimethyl sulfoxide, glycerine, proplylene glycol, isopropyl myristate, ethanol, isopropanol, N-methyl pyrrolidone, dimethyl isosorbide, diisopropyl adipate, benzyl alcohol, medium-chain triglycerides, lecithin.

Suitable oils include, without limitation, almond oil, castor oil, ceratonia extract, cetostearoyl alcohol, cetyl alcohol, cetyl esters wax, cholesterol, cottonseed oil, cyclomethicone, ethylene glycol palmitostearate, glycerin monostearate, glyceryl monooleate, isopropyl myristate, isopropyl palmitate, lanolin, lecithin, light mineral oil, medium-chain triglycerides, mineral oil and lanolin alcohols, petrolatum, petrolatum and lanolin alcohols, soybean oil, starch, stearyl alcohol, olive oil and combinations thereof.

Suitable moisturizers include, but are not limited to, urea, allantoin, glycerine, natural moisturizer factor such as pyrrolidone carboxylic acid

Suitable gelling agents include, but are not limited to, acrylic acid crosspolymer such as Carbopol®, cellulose derivatives such as natrosol®

The emulsion forming agents or emulsifier which may be used in these formulations include carbomers, polyoxyethylene castor oil derivatives, sorbitan fatty acid esters, polyoxyethylene sorbitan fatty acid esters, polyoxyethylene alkyl ethers, caprylic and capric triglycerides, polyethylene glycol esters and others belonging to the class of non-ionic surfactants.

The gelling agents which may be used in these formulations include conventional gelling agents well known for their gelling properties, such as, for example, cellulose ethers such as hydroxypropyl cellulose, hydroxymethyl cellulose, hydroxypropyl methylcellulose, carboxymethyl cellulose, sodium carboxymethylcellulose, hydroxycellulose, and the like; vinyl alcohols; vinyl pyrrolidones; natural gums such as karaya gum, locust bean gum, guar gum, gelan gum, xanthan gum, gum arabic, tragacanth gum, carrageenan, pectin, agar, alginic acid, sodium alginate, chitosan and the like, acrylic acid crosspolymer such as Carbopol® or Eudragit®.

The chelating agents which may be used in these formulations include conventional chelating agents known in the art, such as, for example, disodium edetate.

The formulations may also contain antioxidants, such as, for example, butylated hydroxy anisole (BHA), butylated hydroxy toluene (BHT), tertiary butyl hydroquinone, propyl gallate, α-tocopherol, and the like.

The formulations may further contain preservatives which may be used include phenoxyethanol, methyl paraben, propyl paraben, butyl paraben, benzyl alcohol, and the like.

In accordance with the present invention, the formulations may also contain a pH modifying agent or buffer. The pH modifying agent may be selected from any well known and pharmacologically safe inorganic or organic basic salt. Examples of inorganic basic salts may include magnesium oxide, magnesium hydroxide, calcium hydroxide, sodium hydroxide, potassium hydroxide, potassium carbonate, sodium bicarbonate, and the like. Examples of inorganic acid salts may include hydrochloride, citric acid, acetic acid and the like. Examples of organic basic salts may include methanolanine, ethanolamine, propanolamine, trimethanolamine, triethanolamine, alkylamines such as methylamine, ethylamine, butylamine, isopropylamine, and the like.

### EXAMPLE 1: ANALYSIS OF TOPICAL CLONIDINE ANTI-INFLAMMATORY PROPERTIES IN A HUMAN SKIN ORGANOTYPIC CULTURE MODEL

### I- STUDY DESCRIPTION

### 1- Human skin organotypic culture :

Human biopsy samples of clinically healthy human skin were obtained from patients undergoing plastic surgery. All patients gave their written informed consent before the surgery has carried out.

Skin biopsies were cut into 1 cm2 full thickness pieces and washed three times with antibiotics. Subcutaneous fat and lower dermis were mechanically removed. Skin fragments were transferred onto permeable polycarbonate membrane transwells (12 mm diameter, 12 µm pore size) in a well of 12 well plate (as previously described Boisnic S. et al., [1-3]) containing 700µl of defined medium (DMEM, antibiotics, hormones, pituitary extract, FCS). Explants were maintained at the interface between air and medium with the epithelium uppermost and the dermis exposed to the media by diffusion through the porous membrane.

Cohesion between skin fragments and inserts was obtained with a polyvinylsiloxane addition type silicone elastomer seal (coltène® president) in such a way that no skin retraction or lateral passage of the product towards the dermis was possible.

The culture was set up for 36 hours in a humidified atmosphere containing 5% CO2 at 37°C, and medium was daily change, culture supernatants were frozen at -80°C until further analysis.

At the end of culture all human skin fragments were formalin fixed and paraffin embedded.

At the end of the study, all of the culture supernatants collected unused for cytokine quantification were destroyed.

### 2- Stimulation of skin inflammation in vitro:

The stimulation of the experimental oral human skin inflammatory process was done by the neuropeptide substance P (SP) [1].

The neuropetide SP ([SP] = 10 or 5 µM, was added to the culture medium in order to induce pro-inflammatory process. SP was in contact with the dermis after diffusion through the porous membrane.

### 3- Measure of clonidine anti-inflammatory properties in vitro:

In order to evaluate the preventive effect of clonidine, 200 mg (∼200µl or 0.2 cm3) of clonidine topical formulations was added apically on the epidermis 6 times a day, every 2 hours for 2 days. Ten hours after the first topical clonidine treatment (and in the same time than the last clonidine treatment) substance P was added to the culture medium in the basal compartment.
5 Topical formulations, containing different concentrations of clonidine, were tested: 0%, 0.01 %, 0.05%, 0.1%, 0.5% and 1% of clonidine.

24h after inflammatory process induction:
- Supernatants were collected, aliquoted and stored at -80°C for pro-inflammatory cytokine release analysis: production of TNFα, was measured by enzyme-linked immunosorbent assay (ELISA) in the culture supernatant.
- Skin fragments were fixed and paraffin embedded for histological evaluation of inflammatory reaction parameters, as oedema and vasodilatation. Histological evaluation was performed on the papillary dermis and on the upper part of the reticular dermis, at immediate perivascular localization.

### II- STUDY PROTOCOL

In agreement with the experimental conditions described above, 5 concentrations of clonidine: 0%, 0.05%, 0.1%, 0.5% and 1% were tested on 8 different human skin fragments (cultures SP+).

As controls:
- 8 different fragments were maintained in culture without substance P stimulation and application of topical formulation 0% and 1% clonidine (Culture C-).
- 8 different fragments were tested with clonidine 10⁻⁵M added to the culture medium 10h prior substance P was added (Culture C+).

Day 0:
Biopsy samples of clinically normal human skin were dissected, weighted and
transferred onto permeable polycarbonate membrane transwells.

Clonidine treatment started:
- by applying 200µl of clonidine topic formulations on the epidermis. Clonidine concentrations were as followed: 0%, 0.05%, 0.1%, 0.5% and 1% of clonidine for Cultures SP+.
- by added clonidine solution to culture medium of Culture C+ in order to obtain a final concentration of 10⁻⁵ M (2.3x10⁻⁵ µg/ml).

For Culture C-, start application of topical formulation 0% and 1% clonidine.

The clonidine treatments were repeated every two hours during the day on the apical side of explants culture

Day 0 + 10h:
The clonidine treatments were repeated on the apical side of explants culture.
Substance P was added in culture medium at the basal side of Cultures SP+.
Addition of substance P and clonidine in culture medium of Culture C+.

Day1:
The clonidine treatments were repeated every two hours during the day on the apical side of explants culture

Day1 + 10h: 24h after SP addition

Collect D1 culture medium of each well and aliquot them in 7 samples of 100µl each and store the samples at -80°C for cytokines release analysis.

Skin fragments were removed from insert, fixed in Bouin's liquid and embedded in paraffin for histological evaluation.

### III- STUDY RESULTS

This experiment has been done to measure the preventive effect of clonidine against skin inflammation.

Therefore the clonidine treatment is done before and during substance P stimulation.

The results show that proinflammatory cytokines TNFα level decrease, as well as vasodilatation and skin oedema.

### EXAMPLE 2: TOPICAL GEL

| **Ingredients** | **% by weight** |
|---|---|
| Clonidine | 1 |
| Propylene glycol | 20 |
| Allantoin | 5 |
| Carbomer | 1 |
| Methylparaben | 0.1 |
| Propylparaben | 0.1 |
| Water | q.s. to 100 |
| Triethanolamine | a sufficient amount to adjust pH 3.5-6 |

### EXAMPLE 3: TOPICAL EMULSION

| **Ingredients** | **% by weight** |
|---|---|
| Clonidine | 1 |
| Water | 70.8 |
| Acrylates/C10-30 Alkyl Acrylate Crosspolymer | 0.5 |
| Isopropyl myristate | 13 |
| Mygliol 812 | 14 |
| Triethanolamine | 0.5 |
| Methyl paraben | 0.1 |
| Propyl paraben | 0.1 |

### EXAMPLE 4 OILY DISPERSION

| **Ingredients** | **% by weight** |
|---|---|
| Clonidine | 1 |
| Lanolin | 50% |
| Paraffin oil | 49% |

## Claims

1. Clonidine or its derivatives for the treatment of Hand-Foot Syndrome or related pathologies.

2. Clonidine or its derivatives according to claim 1 in the form of a base.

3. Clonidine or its derivatives according to claim 1 in the form of a pharmaceutically acceptable salt thereof.

4. Clonidine or its derivatives according to claim 3 in the form of clonidine hydrochloride.

5. Clonidine or its derivatives according to anyone of claim 1 to 4 for the prevention of Hand-Foot Syndrome, Hand-Foot Skin Reaction or Palmar Erythema.

6. Clonidine or its derivatives according to anyone of claim 1 to 5 associated with other active ingredient selected from the group consisting of TP inhibitors, DPD inhibitors, anti-inflammatories, vasoconstrictors, differentiation agents, antiangiogenics or antiperspirant.

7. Clonidine or its derivatives according to anyone of claim 1 to 6 wherein the TP inhibitor is selected from 6-substituted 5-fluorouracil derivatives or allopurinol.

8. Clonidine or its derivatives according to anyone of claim 1 to 6 wherein the DPD inhibitor is selected from uracil or uracil derivatives, such as eniluracil.

9. Clonidine or its derivatives according to anyone of claim 1 to 6 wherein the anti-inflammatory is selected from , cyclo-oxygenase-2 Inhibitors as celecoxib, indomethacin and ibuprofen; corticosteroids as dexamethasone, clobetasol 17 propionate, betamethasone.

10. Clonidine or its derivatives according to anyone of claim 1 to 6 wherein the differentiation agent is selected from retinoids, vitamin D or its derivatives, glitazones or fibrates.

11. Clonidine or its derivatives according to anyone of claim 1 to 6 wherein the vasoconstrictor is selected from nicotine or caffeine.

12. Clonidine or its derivatives according to anyone of claim 1 to 6 wherein the antiperspirant is selected from aluminium or zirconium salts, anticholinergics such as glycopyrrolate or carbonic anhydrase inhibitors such as topiramate.

13. Clonidine or its derivatives according to anyone of claim 1 to 6 wherein the antiangiogenics is selected from tacrolimus, pimecrolimus or anti-VEGF.

14. A pharmaceutical composition comprising clonidine or its derivatives according to anyone of claim 1 to 5, for the treatment of Hand-Foot Syndrome or related pathologies.

15. A pharmaceutical composition according to claim 14 wherein clonidine or its derivatives is associated with anyone of other active ingredient as defined in claims 6 to 13.
